# EUROPEAN PATENT SPECIFICATION

(11) **EP 2 422 838 B1**
(45) Date of publication and mention of the grant of the patent: **13.03.2013**
(21) Application number: 11178765.1
(22) Date of filing: 25.08.2011
(51) Int. Cl.: A61M 39/02

(54) **Implantable central venous port**
Implantierbarer zentralvenöser Port
Port veineux central implantable

(30) Priority: 26.08.2010 JP 2010189227
(43) Date of publication of application: 29.02.2012
(73) Proprietor: Covidien LP, Mansfield, MA 02048 (US)
(72) Inventor: Katsumasa Takagi, Fukuroi-shi Shizuoka 437-0004 (JP)
(74) Representative: Gray, James

(56) References cited:
- EP-A2- 1 832 252
- WO-A1-00/33901
- WO-A2-02/074381
- DE-U1-202005 013 295

## Description

### BACKGROUND

### Technical Field

The present disclosure relates to a subcutaneous implantable port used in a subcutaneous implanted state in a patient, and to a method of manufacturing the same.

### Description of Related Art

The conventional use of medical devices known as subcutaneous implantable ports for the long-term delivery of anti-cancer drugs and nutrients to a patient is well known. Subcutaneous implantable ports are connected to intravascular catheters, and are used in a subcutaneously implanted state in the breast region or the like of a patient. Conventional subcutaneous implantable ports normally include a structure in which a septum having an elastic body is disposed in a housing made of a synthetic resin. The housing is configured from a closed bottom cup-shaped base, and a cover that covers this base. The portion of the housing below the septum defines an injection fluid chamber. In addition, an injection needle used to pierce the skin is inserted into the septum, and its tip extends into the injection fluid chamber. The introduction of a quantity of drug in this state results in the intravascular injection of the drug by way of the catheter.

In certain instances, a subcutaneous implantable port may include a metal bottom plate that is provided on the bottom portion of the housing to prevent the injection needle from penetrating the bottom portion of the housing. The bottom plate is, for example, disposed on the bottom portion of a base and fixed thereto by thermal bonding. In some instances, a retaining lip may be integrally pre-moulded in the base to facilitate coupling the bottom plate to the bottom portion. See Japanese Laid-Open Patent Application 2007-643.

EP1832252, DE202005013295, WO02/074381 and WO00/33901 all disclose prior art type ports where the bottom plate is fixed to the port housing by, for example, threaded connections, and mating male and female connections.

Problems arise in the conventional art described above. In the example of the conventional art in which the bottom plate is fixed by thermal welding, the base and the cover are fixed by ultrasonic welding in a step subsequent to a thermal welding step, and the bottom plate - for which oscillation should essentially be avoided - is oscillated by the ultrasonic waves at this time. This causes the welded portion of the perimeter of the bottom plate to melt, and this in turn leads to problems including positional displacement and separation of the bottom plate.

In addition, in the example of the conventional art in which a bottom plate is fixed using a retaining lip, not only is the step for displacing the retaining lip complex to perform, the fixing of the bottom plate is often inadequate.

In addition to the foregoing, other methods including a method based on embedding the bottom plate in the base by insert-molding and a method based on the employment of a separate fixing part to fix the bottom plate to the base have been considered. An unavoidable complexity and increased manufacturing costs are inherent in the steps involved in each of these methods.

In view of the foregoing, it may prove advantageous to provide a subcutaneous implantable port in which the bottom plate for preventing penetration of the housing by the injection needle can be reliably fixed. Moreover, it may prove advantageous to provide a method that allows the improved subcutaneous implantable port described above to be comparatively more easily manufactured.

### SUMMARY

The present disclosure provides a subcutaneous implantable port which includes a housing having a structure in which a closed bottom cup-shaped base with a recess portion that defines an injection fluid chamber is covered by a cover. A septum configured for insertion by an injection needle is disposed within the housing in a state in which a portion thereof is exposed through a hole portion in the cover. A bottom plate made of a material harder than the aforementioned base is disposed on the inner-surface side of a bottom portion of the base. A catheter connecting portion is disposed on the outer surface of the housing. The bottom plate has a plurality of corner portions on its outer peripheral edge portion. The bottom plate is fixed to the bottom portion of the base as a result of the plurality of corner portions biting into the inner surface of the recess portion from the circumferential direction of the bottom plate.

Accordingly, as a result of the plurality of corner portions of the bottom plate biting into the inner surface of the recess portion from the circumferential direction of the bottom plate, the bottom plate is reliably fixed to the bottom portion of the base without need for a retaining lip or separate fixing part.

In certain embodiments, the thickness of the outer peripheral edge portion of the bottom plate in which the plurality of corner portions are formed is less than the thickness toward the center portion of the bottom plate.

Accordingly, when the plurality of corner portions of the bottom plate bite into the inner surface of the recess portion from the circumferential direction of the bottom plate, the resistance to the plurality of corner portions is less. For this reason, the plurality of corner portions can be readily and reliably caused to bite into the inner surface of the recess portion. Consequently, the extent to which the corner portions bite into the inner surface of the recess portion can be increased and, in turn, a more reliable fixing of the bottom plate can be achieved.

In embodiments, the bottom plate defines a polygonal shape having five or more corner portions. Because the number of positions at which the corner portions bite into the inner surface of the recess portion is comparatively greater than if a triangular or a square bottom plate is employed, the bottom plate can be more reliably fixed. Notably, the polygonal shape noted above is preferably a regular polygon which, in this case, is effective from the standpoint of ensuring evenness of the extent of the bite of the corner portions.

The present disclosure also provides a method for manufacturing the subcutaneous implantable port. The method includes the following steps: fixing the bottom plate to the bottom portion of the base by rotating the base plate in the circumferential direction thereof so as to cause the plurality of corner portions to bite into the inner surface of the recess portion and ultrasonic welding the bottom plate by applying ultrasonic waves to weld the base to the cover.

Accordingly, when the base plate is rotated in the circumferential direction thereof, the plurality of corner portions are caused to bite into the inner surface of the recess portion, and the bottom plate is fixed to the bottom portion of the base. Thereafter, when ultrasonic waves are applied in the ultrasonic welding step, the base and cover are welded to form a single piece to form a housing. Accordingly, based on this method of manufacture, because of the absence of any need to implement a special step or increase the number of component parts to fix the bottom plate, the improved subcutaneous implantable port as described above can be comparatively more easily manufactured.

In embodiments, the method for manufacturing a subcutaneous implantable port wherein the bottom plate is simultaneously welded and fixed to the base in the ultrasonic welding step.

Accordingly, when ultrasonic waves are applied in the ultrasonic welding step, not only is the boundary portion between the base and the cover fused and welded, the portion of the base plate bitten into by the corner portions is also oscillated and caused to generate heat. As a result of the melting of the material surrounding the section bitten into by the corner portions and the resulting formation of a raised portion that covers the corner portions of the bottom plate, the bottom plate is reliably fixed by welding to the base. Consequently, an increase in the number of steps can be avoided.

In embodiments, in the bottom plate fixing step of the method for manufacturing a subcutaneous implantable port, a bottom plate in which a jig engaging portion has been pre-formed is employed, and the bottom plate is rotated in the circumferential direction thereof as a result of a rotational force imparted thereto by the jig in a state in which the distal end of the jig is engaged with the jig engaging portion.

Because a rotational force is imparted to the bottom plate by a jig in a state in which the distal end of the jig is coupled to the jig engaging portion, the bottom plate can be rotated in the circumferential direction with a comparatively small force. For this reason, the corner portions of the bottom plate can be easily and reliably caused to bite into the inner surface of the recess portion.

Accordingly, a subcutaneous implantable port in which the bottom plate for preventing the penetration of the housing by the injection needle can be reliably fixed with no associated increase in costs can be provided. In addition, the method allows for the improved subcutaneous implantable port described above to be comparatively more easily manufactured.

### BRIEF DESCRIPTION OF THE DRAWINGS

Various embodiments of the present disclosure are described herein below with references to the drawings, wherein:
FIG. 1a is a front view of a subcutaneous implantable port according to an embodiment of the present disclosure;
FIG. 1b is a plan view of the subcutaneous implantable port depicted in FIG. 1a;
FIG. 2 is a cross-sectional view along the line A-A of FIG. 1b;
FIG. 3a is a plan view of a bottom plate of the subcutaneous implantable port depicted in FIG. 2;
FIG. 3b is a cross-sectional view taken along section line B-B of FIG. 3a illustrating the bottom plate of FIG. 3a;
FIG. 3c is a plan view of the base prior to fixing the bottom plate thereto;
FIG. 4 is a cross-sectional view illustrating the bottom plate positioned on a bottom portion of the base in a pre-affixed configuration;
FIG. 5 is a plan view showing the bottom plate positioned on the bottom portion of the base as the bottom plate is rotated into the affixed configuration;
FIG. 6 is a plan view illustrating the bottom plate and the bottom portion in an affixed configuration;
FIG. 7 is an expanded plan view illustrating a portion of the bottom plate and the bottom portion in the affixed configuration;
FIG. 8a is an expanded cross-sectional view of the main portion (region P1 of FIG. 4) showing a state prior to an ultrasonic welding step according to an embodiment of the instant disclosure;
FIG. 8b is an expanded cross-sectional view of the main portion showing the state thereof following ultrasonic welding;
Fig. 8c is an expanded cross-sectional view of the main portion showing a state thereof following ultrasonic welding in an example of the prior art; and
FIGS. 9a-9f are plan views of bottom plates according to various embodiments of the present disclosure.

### DETAILED DESCRIPTION

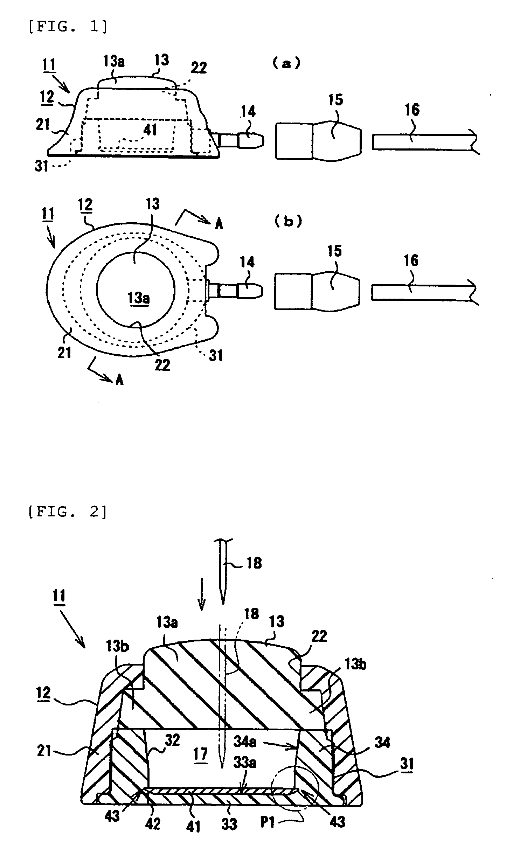

The subcutaneous implantable port of a specific embodiment of the present invention will be hereinafter described in detail with reference to FIGS. 1 to 9.

A subcutaneous implantable port 11 of the embodiment shown in FIGS. 1 and 2 and so on is of the type used for the long-term delivery of anti-cancer drugs and nutrients to a patient. The housing 12 from which the subcutaneous implantable port 11 is configured is formed from a base 31 and a cover 21. The base 31 is a molded article formed using a general purpose synthetic resin such as polypropylene and, as shown in FIG. 2, comprises a bottom portion 33 and a side wall 34 that extends upward from the outer circumferential portion of the bottom portion 33. The upper end of the side wall 34 is open. A flange is formed in the lower-end outer peripheral portion of the side wall 34. A tubular protruding portion 36 enclosing a catheter connecting portion 14 is provided in a portion of the flange. The base 31 describes an overall closed bottom cup-shape, and comprises a recess portion 32 that defines an injection fluid chamber 17 within the side wall 34.

Cover 21 is formed to be slightly larger than the base 31, and a circular hole portion 22 is formed in a center portion of the upper surface of the cover 21. Similarly to the base 31, this cover 21 also constitutes a molded article formed from a general purpose synthetic resin such as propylene. The cover 21 is affixed to the base 31 in such a way as to cover the base 31 from above, and the cover and the base are fixed to each other by ultrasonic welding. Notably, while the cover 21 and the base 31 are fixed to each other in this embodiment by ultrasonic welding, they may be fixed to each other by adhesion, or by fit-insertion or the like.

As is shown in FIG. 2 and so on, a septum 13 is made from an elastic body (preferably a silicone resin) such as, for example, a hard rubber and is disposed within the housing 12. The septum 13, which constitutes a member that defines a circular shape in the planar view, has a thickly-formed center portion 13a that distends toward the upper surface of the cover 21. An outer periphery portion 13b of the septum 13 is sandwiched and fixed between the upper-end portion of the side wall 34 of the base 31 and the outer periphery portion of the upper-end rear surface side of the cover 21. An injection needle 18 that protrudes to the exterior by way of the hole portion 22 of the cover 21 is able to be inserted through the thick center portion 13a of the septum 13. Notably, the septum 13 blocks off injection fluid chamber 17 in such a way as to prevent leakage of the drug or the like from the injection fluid chamber 17.

A predetermined location of the outer peripheral portion of the cover 21 is formed in a recessed shape, and the catheter connecting portion 14 is arranged in this section. The catheter connecting portion 14 passes through the side wall 34 of the base 31 to afford communication between the recess portion 32 and the external region of the base 31. A synthetic resin catheter fixing device 15 is provided in the connecting end side of the catheter 16. In addition, as a result of the affixing of the catheter fixing device 15 to the catheter connecting portion 14 in a state in which the catheter 16 is inserted in the catheter connecting portion 14, the catheter 16 is reliably fixed to the catheter connecting portion 14.

The bottom plate fixing structure of the subcutaneous implantable port 11 of this embodiment will be hereinafter described.

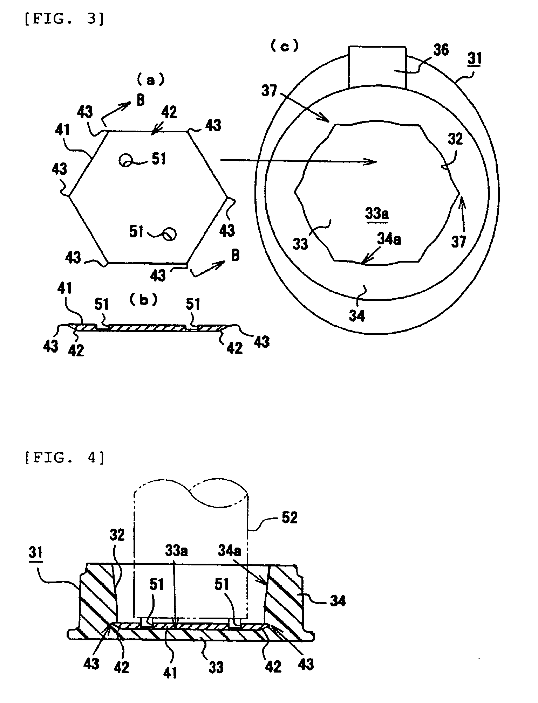

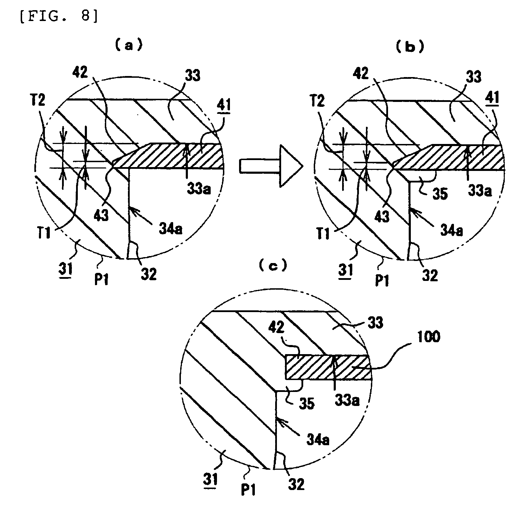

As shown in FIGS. 3a and 3b, the subcutaneous implantable port 11 includes a bottom plate 41. The bottom plate 41 of this embodiment constitutes a member made of a metal, for example, titanium, harder than the base 31, cover 21 and septum 13. In one embodiment, the bottom plate 41 defines a regular hexagon shape in the planar view, and comprises six corner portions 43 in its outer peripheral edge portion 42. The outer peripheral edge portion 42 of the bottom plate 41 in which the corner portions 43 are provided is formed in a tapered shape as a result of the scarfing of the lower-surface side of the bottom plate 41. Accordingly, a thickness T1 of the outer peripheral edge portion 42 of the bottom plate 41 in which the corner portions 43 are provided is somewhat thinner than the thickness T2 toward the center portion of the bottom plate 41 (See FIG. 3b and FIG. 8a, 8(b). In addition, holes 51 serving as a jig engaging portion are formed in two separate locations in the bottom plate 41. These holes 51 are non-penetratable holes provided only in the upper-surface side of the bottom plate 41. The reason for this is to assist in more reliably preventing penetration of the housing by the injection needle 18.

In addition, as shown in FIG. 3c, the recess portion 32 of the base 31 is formed in a size essentially the same as that of the bottom plate 41 in the planar view, and curved recess portions 37 designed to avoid the corner portions 43 are provided in six locations thereof. Notably, with consideration to ease of mounting the bottom plate 41, the recess portion 32 of this embodiment is slightly larger at the upper-end open side of the side wall 34 (see FIG. 2).

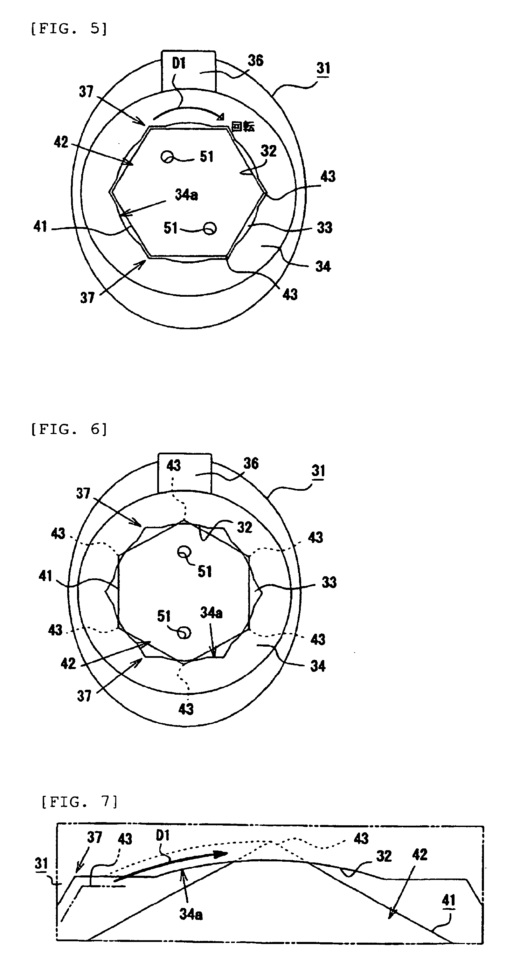

The bottom plate 41 of the structure described above is disposed in such a way as to have surface contact with a bottom portion inner surface 33a side of the base 31. In addition, the six corner portions 43 of the bottom plate 41 bite into the inner surface side 34a of each recess portion 32 from the circumferential direction D1 of the bottom plate 41 (see FIGS. 5 to 7). As a result, the bottom plate 41 is fixed to the bottom portion 33 of the base 31.

A method of manufacturing the subcutaneous implantable port 11 of this embodiment will be hereinafter described.

First, each of the base 31, the cover 21, the septum 13 and the bottom plate 41 and so on are prepared for assembly. Next, the bottom plate 41 is disposed on the bottom portion inner surface 33a side of the base 31. As a result, the bottom surface side (that is to say, the surface side in which there are no holes 51 provided) of the bottom plate 41 is brought into contact with the bottom portion inner surface 33a. In addition, as shown in FIG. 5, the six corner portions 43 are disposed in such a way as to be positioned in the respective curved recess portions 37.

Here, a dedicated jig 52 having two engaging protrusions in its distal end is employed, and these engaging protrusions engage with the two holes 51 of the bottom plate 41. The jig 52 is operated in its engaged state to impart a rotational force which rotates the bottom plate 41 in its circumferential direction D1 by the order of 20 to 40° (more preferably 30°). Thereupon, the corner portions 43 are caused to bite into the inner surface side 34a of the recess portion 32 from the circumferential direction D1 of the bottom plate 41. As a result, a state in which the bottom plate 41 is supported at six points on the outer peripheral edge portion is established, and the bottom plate 41 is reliably fixed to the bottom portion 33 of the base 31 (see FIGS. 6 and 7).

Following this bottom plate fixing step, the septum 13 is arranged in such a way as to be sandwiched between the base 31 and the cover 21, and the ultrasonic welding step is implemented. As a result of the application of ultrasonic waves on the interface portion between the base 31 and the cover 21 in the implementation of this step, the base 31 and the cover 21 are welded to form a single piece to produce the housing 12. Because the ultrasonic waves are simultaneously applied to the bottom plate 41 at this time, the corner portions 43 of the bottom plate 41 that are being caused to bite into the base 31 side are also oscillated to generate heat. Thereupon, the resin material surrounding the corner portions 43 is melted by the heat generated thereby, and a raised portion 35 that covers these corner portions 43 is formed (see FIGS. 8a and 8b). As a result, a state in which the bottom plate 41 is more reliably welded and fixed to the base 31 is established. Incidentally, FIG. 8c shows the state following the ultrasonic wave welding step of the prior art. The bottom plate 100 of the example of the prior art does not have special corner portions and, as such, does not constitute a structure in which the outer peripheral edge portion 42 of the bottom plate 100 bites into the inner surface side 34a of the base 31. To put this another way, the outer peripheral edge portion 42 of the bottom plate 100 does not extend to the inner surface side 34a, and is instead positioned more toward the center of the recess portion 32 from the inner surface side 34a.

The usage method of the subcutaneous implantable port 11 of this embodiment will be hereinafter briefly described.

First, as the preparation for implantation, a wide area of the skin around the intended area for implantation of the subcutaneous implantable port 11 and the catheter 16 is disinfected. Next, a local or a general anaesthetic is administered by normal methods, and the catheter 16 is implanted in the target area by incision method or the Seldinger technique or the like. The injection needle 18 is inserted into the septum 13 and the injection fluid chamber 17 is filled with a heparin-added physiological saline solution and, as a result, the air is discharged fully therefrom. Thereafter, an incision is made in the skin, and a subcutaneous pocket is produced in the area intended for implantation of the subcutaneous implantable port 11. The connecting end of the catheter 16 is cut to a suitable length, and the catheter fixing device 15 is inserted into the catheter 16. Thereafter, the catheter 16 is firmly inserted in the catheter connecting portion 14 of the subcutaneous implantable port 11 and, in this state, the catheter fixing device 15 is reliably affixed. In this state, the injection needle 18 is punctured through the septum 13, the heparin-added physiological saline solution is injected and, in a smooth flow, the absence of any liquid leak is confirmed. Finally, the subcutaneous impiantable port 11 is arranged in the subcutaneous pocket and fixed therein by sewing to the fascia or the like, and the incision is then sutured to complete the operation for the implantation of the catheter 16 and the subcutaneous implantable port 11.

The injection of a drug or the like may be implemented for a period of around 1 week following the implantation of the catheter 16 and the subcutaneous implantable port 11. First, the skin surrounding the section where the subcutaneous implantable port 11 is implanted is thoroughly disinfected. The injection needle 18 is percutaneously punctured through the surface center portion of the subcutaneous implantable port 11. More specifically, the tip of injection needle 18 is extended through septum 13 to reach the injection fluid chamber 17 of the housing 12. In this embodiment, because of the hard metal bottom plate 41 disposed in the bottom portion inner surface 33a of the housing 12, even if the tip of the injection needle 18 collides with the bottom plate 41, a state in which the bottom portion 33 is penetrated thereby can be prevented. In this state, following the injection of several mL of heparin-added physiological solution and confirmation that the catheter 16 is open, a drug such as an anti-cancer agent is intravascularly injected. Upon completion of the delivery of the drug, several mL of heparin-added physiological solution (heparin lock) is injected and, taking care to avoid backflow to the catheter 16, the needle is withdrawn. This completes the injection operation.

The following actions and effects are afforded by the embodiment described above.

In the subcutaneous implantable port 11 of this embodiment, because the bottom plate 41 is disposed on the bottom portion inner surface 33a side of the base 31 from which the housing 12 is constituted, a state in which the top of the injection needle 18 penetrates the bottom portion 33 of the housing can be prevented. In addition, a state in which the six corner portions 43 of the bottom plate 41 bite firmly into the inner surface side 34a of the recess portion 32 form the circumferential direction D1 of the bottom plate 41 is established. Consequently, even when some degree of melting of the resin surrounding the bottom plate 41 occurs in the ultrasonic welding step, positional displacement or separation of the bottom plate 41 is avoided and, as such, added reliability is achieved. In addition, because the need for the retaining lip or a separated fixing part of the prior art is eliminated, the bottom plate 41 can be reliably fixed to the bottom portion 33 of the base 31 with no associated increase in costs and in the absence of any need to implement a complex manufacturing step.

The thickness T1 of the outer peripheral edge portion 42 of the bottom plate 41 in which the six corner portions 43 are provided is less than the thickness T2 of the center portion side of the bottom plate 41. As such, when the corner portions 43 bite into the inner surface side 34a of the recess portion 32 from the circumferential direction D1 of the bottom plate 41, there is comparatively less resistance at the corner portions 43. Accordingly, the corner portions 43 can be readily and reliably caused to bite into the inner surface side of the recess portion. Consequently, the extent to which the corner portions 43 are able bite into the inner surface side of the recess portion can be increased, and the bottom plate 41 can be more reliably fixed.

In one embodiment, the bottom plate 41 constitutes a member that defines a regular hexagon shape in the planar view and, as such, it comprises six corner portions 43. Accordingly, compared to the employment of, for example, a triangular- or square-shaped bottom plate, the number of locations where the corner portions 43 are able to bite into the inner surface side of the recess portion can be increased, and the corner portions 43 can be more reliably fixed to the base 31. Alternatively, other configurations having multiple corner portions are envisioned. In addition, because the bottom plate 41 is hexagonal, rotating the bottom plate 41 at the same angle ensures that the degree to which the corner portions 43 bite into the inner surface side of the recess portion is even.

According to the method of manufacturing the subcutaneous implantable port 11 of this embodiment described above, when the bottom plate 41 is rotated in its circumferential direction D1 in the bottom plate fixing step, the six corner portions 43 bite into the inner surface side 34a of the recess portion 32, and the bottom plate 41 fixes to the bottom portion 33 of the base 31. Thereafter, when ultrasonic waves are applied in the ultrasonic welding step, the base 31 and cover 21 are welded to form a single piece to produce the housing 12. According to this method of the manufacturing, because of the absences of a particular need to increase the number of steps or the number of component parts in order to fix the bottom plate 41, the improved subcutaneous implantable port 11 described above can be comparatively more easily manufactured.

As a result of the application of ultrasonic waves in the ultrasonic welding step of the method of manufacturing of this embodiment, not only is the boundary portion between the base 31 and the cover 21 fused and welded, the biting portions of the bottom plate 41 are also oscillated to generate heat. At this time, the resin material surrounding these biting portions melts, and a raised portion 35 that covers these corner portions 43 of the bottom plate 41 is formed. As a result, the bottom plate 41 is more reliably fixed by welding to the base 31. As a consequence, an increase in the number of steps can be avoided.

In the method of manufacturing of this embodiment, the distal end of the jig 52 is engaged with two holes 51 serving as jig engaging portions, and a rotational force is imparted to the bottom plate 41 by the jig 52 in this state. That is to say, where under original conditions the rotation of the bottom plate 43 would be difficult because of an arrangement of the recess portion 32 in the base portion inner surface 33a, it can be easily rotated in the circumferential direction D1 using a comparatively small force. As such, the corner portions 43 of the bottom plate 41 can be easily and readily caused to bite into the base 31.

Notably, the embodiment of the present invention may be modified in the manner described below.

While the aforementioned embodiment describes a subcutaneous implantable port 11 for the delivery of drugs such as anti-cancer agents, the subcutaneous implantable port 11 may be one that is used for the delivery of, for example, nutrients.

While the aforementioned embodiment describes the use of a metal bottom plate as the bottom plate 41 constituted from a material harder than the base 31, this may replaced by the use of, for example, a ceramic bottom plate. Alternatively, other materials resistant to penetration by needle 18 may be used to construct bottom plate 41.

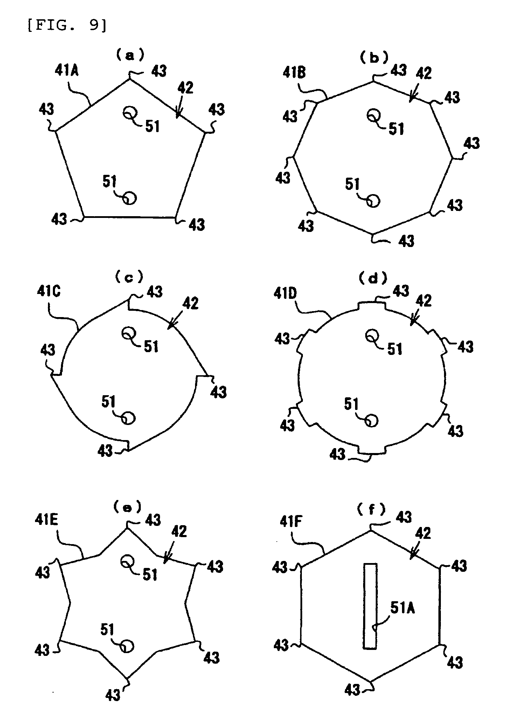

While the aforementioned embodiment describes an example in which a hexagonal bottom plate 41 is used, this is of course not limited thereto. For example, as shown in the separate embodiment of FIG. 9a, a bottom plate 41A of a regular pentagon shape having five corner portions 43 may be used and, as shown in the separate embodiment of FIG. 9b, a bottom plate 41B of a regular octagon shape having eight corner portions 43 may be used.

While the aforementioned embodiments describe examples of bottom plates 41, 41A, 41B of a regular shape, the implementation of the separate embodiments shown in FIGS. 9c to 9e may be adopted. The bottom plate 41C shown in FIG. 9c describes a structure in which corner portions 43 shaped like right-angled triangles protrude from four locations of a circular-shaped bottom plate main body. The bottom plate 41D shown in FIG. 9d describes a structure in which essentially oblong-shaped corner portions 43 protrude from six locations of a square-shaped bottom plate main body. FIG. 9e describes bottom plate 41E with a star-shape structure having six corner portions 43. Notably, each of the embodiments described above define a rotationally symmetrical shape about the center of the bottom plate.

While the aforementioned embodiment describes an example of a bottom plate 41 in which two holes 51 are formed as jig engaging portions, three or more of these holes 51 may be formed. In addition, as in the bottom plate 41F of the separate embodiment shown in FIG. 9f, a groove 51A may be formed as the jig engaging portion.

While the aforementioned embodiment describes an example in which the corner portions 43 are comparatively sharp angles, provided they are sharp enough to be oscillated by the ultrasonic waves of the ultrasonic welding step, these corner portions 43 may define a slightly less sharp angle.

In accordance with the instant disclosure, the shape and size of the bottom plate in the planar view is essentially equivalent to the shape and the size of the recess portion in the planar view and, in addition, curved recess portions for avoiding the corner portions are provided in the inner surface of the recess portion.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. A subcutaneous implantable port (11) comprising:
a housing (12) having a closed base (31) with a recess portion (32) defining an injection fluid chamber (17), the housing (12) having a cover (21) positioned over the base (31);
a septum (13) positioned between the cover (21) and the base (31) adjacent the injection fluid chamber (17), the septum (13) being configured to be penetrated by an injection needle;
a bottom plate (41) disposed on an inner-surface (33a) of a bottom portion of the base (31); and
a catheter connecting portion (14) disposed on the outer surface of the housing (12),
**characterised in that** the bottom plate (41) includes a plurality of corner portions (43) on an outer peripheral edge portion thereof (42), and the bottom plate (41) is fixed to the bottom portion of the base (31) as a result of the plurality of corner portions (43) biting into an inner surface side (34a) of the base (31) defining the recess portion (32) from the circumferential direction of the bottom plate (41).

2. A subcutaneous implantable port (11) according to claim 1, wherein the thickness (T1) of the outer peripheral edge portion (42) of the bottom plate (41) in which the plurality of corner portions (43) are formed is less than the thickness (T2) of the bottom plate (41) toward a center portion of the bottom plate (41).

3. A subcutaneous implantable port (11) according to claim 1, wherein the bottom plate (41) defines a polygonal shape having at least five corner portions (43).

4. A subcutaneous implantable port (11) according to claim 1, wherein the bottom plate (41) defines a shape selected from the group consisting of a rectangular pentagon (41A) and rectangular octagon (41B).

5. A subcutaneous implantable port (11) according to claim 1, wherein the bottom plate (41) defines a circular shape (41C) with four right-angled corner portions (43) extending therefrom.

6. A subcutaneous implantable port (11) according to claim 1, wherein the bottom plate (41) defines a square shape having six oblong corner portions (43) extending therefrom.

7. A subcutaneous implantable port (11) according to claim 1, wherein the bottom plate (41) defines a star shape (41E) having six corner portions (43) extending therefrom.

8. A subcutaneous implantable port (11) according to claim 1, wherein the bottom plate (41) is made of a material harder than the base (31).

9. A subcutaneous implantable port (11) according to claim 1, wherein the bottom plate (41) includes at least one pre-formed jig engaging portion that is configured to receive a corresponding jig (52) to facilitate rotation of the bottom plate (41) with respect to the bottom portion of the base (31).

10. A subcutaneous implantable port (11) according to claim 9, wherein the at least one pre-formed jig is in the form of one of at least three holes (51) and a groove (51A).

11. A method for manufacturing a subcutaneous implantable port (11) comprising:
fixing a bottom plate (41) to a bottom portion of a base (31) of the subcutaneous implantable port (11) by rotating the bottom plate (41) in a circumferential direction thereof so as to cause a plurality of corner portions (43) of the bottom plate (41) to bite into an inner surface side (34a) of a recess portion (32) of the base (31);
positioning a septum (13) within the base (31); and
ultrasonically welding a cover (21) to the base of the subcutaneous implantable port (11) to secure the septum (13) within the base (31).

12. A method according to claim 11, wherein the bottom plate (41) is simultaneously welded and fixed to the base (31) in the ultrasonic welding step.

13. A method according to claim 11, wherein in the fixing step the bottom plate (41) is provided with pre-formed jig engaging portion (51, 51A) that is employed to facilitate rotation of the bottom plate (41) in the circumferential direction thereof as a result of a rotational force imparted thereto by a jig (52) engaged with the jig engaging portion (51, 51A).

## Patentansprüche

1. Subkutaner implantierbarer Port (11), der Folgendes umfasst:
ein Gehäuse (12) mit einer geschlossenen Basis (31) mit einem Vertiefungsabschnitt (32), der eine Injektionsflüssigkeitskammer (17) definiert, wobei das Gehäuse (12) eine über der Basis (31) angeordnete Abdeckung (21) aufweist;
ein zwischen der Abdeckung (21) und der Basis (31) neben der Injektionsflüssigkeitskammer (17) angeordnetes Septum (13), wobei das Septum (13) so konfiguriert ist, dass es von einer Injektionsnadel penetriert werden kann;
eine auf einer Innenseite (33a) eines unteren Abschnitts der Basis (31) angeordnete Bodenplatte (41); und
ein auf der Außenseite des Gehäuses (12) angeordneter Katheterverbindungsabschnitt (14);
**dadurch gekennzeichnet, dass** die Bodenplatte (41) eine Vielzahl von Eckabschnitten (43) auf einem äußeren Umfangsrandabschnitt (42) davon aufweist und die Bodenplatte (41) am unteren Abschnitt der Basis (31) durch eine Vielzahl von Eckabschnitten (43), die in eine Seite der Innenseite (34a) der Basis (31) eingreifen und den Vertiefungsabschnitt (32) von der Umfangsrichtung der Bodenplatte (41) definieren, befestigt ist.

2. Subkutaner implantierbarer Port (11) nach Anspruch 1, worin die Dicke (T1) des äußeren Umfangsrandabschnitts (42) der Bodenplatte (41), in der die Vielzahl von Eckabschnitten (43) geformt ist, kleiner ist als die Dicke (T2) der Bodenplatte (41) zu einem mittleren Abschnitt der Bodenplatte (41) hin.

3. Subkutaner implantierbarer Port (11) nach Anspruch 1, worin die Bodenplatte (41) eine polygonale Gestalt mit zumindest fünf Eckabschnitten (43) definiert.

4. Subkutaner implantierbarer Port (11) nach Anspruch 1, worin die Bodenplatte (41) eine Gestalt definiert, die aus der aus einem rechteckigen Pentagon (41A) und einem rechteckigen Oktagon (41B) bestehenden Gruppe ausgewählt ist.

5. Subkutaner implantierbarer Port (11) nach Anspruch 1, worin die Bodenplatte (41) eine kreisförmige Gestalt (41C) mit vier sich von ihr erstreckenden rechtwinkligen Eckabschnitten (43) definiert.

6. Subkutaner implantierbarer Port (11) nach Anspruch 1, worin die Bodenplatte (41) eine quadratische Gestalt mit sechs sich von ihr erstreckenden länglichen Eckabschnitten (43) definiert.

7. Subkutaner implantierbarer Port (11) nach Anspruch 1, worin die Bodenplatte (41) eine sternförmige Gestalt (41E) mit sechs sich von ihr erstreckenden Eckabschnitten (43) definiert.

8. Subkutaner implantierbarer Port (11) nach Anspruch 1, worin die Bodenplatte (41) aus einem Material besteht, das härter als die Basis (31) ist.

9. Subkutaner implantierbarer Port (11) nach Anspruch 1, worin die Bodenplatte (41) zumindest einen vorgeformten, in eine Einspannvorrichtung eingreifenden Abschnitt aufweist, der so konfiguriert ist, dass er eine entsprechende Einspannvorrichtung (52) zur Erleichterung der Drehung der Bodenplatte (41) relativ zu der Bodenplatte der Basis (31) aufnehmen kann.

10. Subkutaner implantierbarer Port (11) nach Anspruch 9, worin die zumindest eine vorgeformte Einspannvorrichtung in Form von einem von zumindest drei Löchern (51) und einer Kerbe (51A) vorliegt.

11. Verfahren zur Herstellung eines subkutanen implantierbaren Ports (11), das Folgendes umfasst:
Befestigung einer Bodenplatte (41) an einem unteren Abschnitt einer Basis (31) des subkutanen implantierbaren Ports (11) durch Drehen der Bodenplatte (41) in einer Umfangsrichtung davon, um zu verursachen, dass eine Vielzahl von Eckabschnitten (43) der Bodenplatte (41) in eine Seite (34a) der Innenseite eines Vertiefungsabschnitts (32) der Basis (31) eingreift;
Positionierung eines Septums (13) in der Basis (31); und
Ultraschallschweißen einer Abdeckung (21) am Boden des subkutanen implantierbaren Ports (11) zur Befestigung des Septums (13) in der Basis (31).

12. Verfahren nach Anspruch 11, worin die Bodenplatte (41) in dem Ultraschallschweißschritt gleichzeitig an der Basis (31) angeschweißt und befestigt wird.

13. Verfahren nach Anspruch 11, worin der Befestigungsschritt der Bodenplatte (41) mit einem vorgeformten in eine Einspannvorrichtung eingreifenden Abschnitt (51, 51A) versehen ist, der zur Erleichterung der Drehung der Bodenplatte (41) in Umfangsrichtung davon durch eine von einer Einspannvorrichtung (52), die mit dem mit der Einspannvorrichtung in Eingriff stehenden Abschnitt (51, 51A) in Eingriff steht, aufgebrachte Drehkraft verwendet wird.

## Revendications

1. Chambre implantable sous-cutanée (11) comprenant :
un boîtier (12) ayant une base fermée (31) avec une partie en évidement (32) définissant une chambre (17) de fluide d'injection, le boîtier (12) ayant un couvercle (21) positionné au-dessus de la base (31) ;
un septum (13) positionné entre le couvercle (21) et la base (31) de manière adjacente à la chambre (17) de fluide d'injection, le septum (13) étant configuré de façon à être pénétré par une aiguille d'injection ;
une plaque inférieure (41) disposée sur une surface interne (33a) d'une partie inférieure de la base (31) ; et
une partie (14) de raccordement de cathéter disposée sur la surface externe du boîtier (12),
**caractérisée en ce que** la plaque inférieure (41) comporte une pluralité de parties de coin (43) sur sa partie (42) de bord périphérique externe, et la plaque inférieure (41) est fixée à la partie inférieure de la base (31) après que la pluralité de parties de coin (43) a mordu dans un côté (34a) de surface interne de la base (31) définissant la partie en évidement (32) depuis la direction circonférentielle de la plaque inférieure (41).

2. Chambre implantable sous-cutanée (11) selon la revendication 1, dans laquelle l'épaisseur (T1) de la partie (42) de bord périphérique externe de la plaque inférieure (41), où la pluralité de parties de coin (43) sont formées, est inférieure à l'épaisseur (T2) de la plaque inférieure (41) vers une partie centrale de la plaque inférieure (41).

3. Chambre implantable sous-cutanée (11) selon la revendication 1, dans laquelle la plaque inférieure (41) définit une forme polygonale ayant au moins cinq parties de coin (43).

4. Chambre implantable sous-cutanée (11) selon la revendication 1, dans laquelle la plaque inférieure (41) définit une forme choisie dans le groupe constitué d'un pentagone rectangulaire (41A) et d'un octogone rectangulaire (41B).

5. Chambre implantable sous-cutanée (11) selon la revendication 1, dans laquelle la plaque inférieure (41) définit une forme circulaire (41C) ayant quatre parties de coin (43) à angle droit qui s'en étendent.

6. Chambre implantable sous-cutanée (11) selon la revendication 1, dans laquelle la plaque inférieure (41) définit une forme carrée ayant six parties de coin (43) de forme oblongue qui s'en étendent.

7. Chambre implantable sous-cutanée (11) selon la revendication 1, dans laquelle la plaque inférieure (41) définit une forme en étoile (41E) ayant six parties de coin (43) qui s'en étendent.

8. Chambre implantable sous-cutanée (11) selon la revendication 1, dans laquelle la plaque inférieure (41) est réalisée en un matériau plus dur que la base (31).

9. Chambre implantable sous-cutanée (11) selon la revendication 1, dans laquelle la plaque inférieure (41) comporte au moins une partie d'engagement de gabarit préformé qui est configurée pour recevoir un gabarit correspondant (52) afin de faciliter la rotation de la plaque inférieure (41) par rapport à la partie inférieure de la base (31).

10. Chambre implantable sous-cutanée (11) selon la revendication 9, dans laquelle l'au moins un gabarit préformé est sous la forme d'un d'au moins trois trous (51) et d'une rainure (51A).

11. Procédé de fabrication d'une chambre implantable sous-cutanée (11), comprenant le fait :
de fixer une plaque inférieure (41) à une partie inférieure d'une base (31) de la chambre implantable sous-cutanée (11) en faisant tourner la plaque inférieure (41) dans sa direction circonférentielle de manière à amener une pluralité de parties de coin (43) de la plaque inférieure (41) à mordre dans un côté (34a) de la surface interne d'une partie en évidement (32) de la base (31) ;
de positionner un septum (13) à l'intérieur de la base (31) ; et
de souder par ultrasons un couvercle (21) à la base de la chambre implantable sous-cutanée (11) pour fixer le septum (13) à l'intérieur de la base (31).

12. Procédé selon la revendication 11, dans lequel la plaque inférieure (41) est simultanément soudée et fixée à la base (31) dans l'étape de soudage par ultrasons.

13. Procédé selon la revendication 11, dans lequel, dans l'étape de fixation, la plaque inférieure (41) est pourvue d'une partie (51, 51A) d'engagement de gabarit préformé qui est utilisée pour faciliter la rotation de la plaque inférieure (41) dans sa direction circonférentielle à la suite d'une force de rotation qui lui est transmise par un gabarit (52) en prise avec la partie (51,51A) d'engagement de gabarit.
